# EUROPEAN PATENT APPLICATION

(11) **EP 1 938 838 A1**
(43) Date of publication of application: **02.07.2008**
(21) Application number: 06810290.4
(22) Date of filing: 13.09.2006
(51) Int. Cl.: A61K 45/00, A61K 31/417, A61K 31/4178, A61P 1/16, A61P 3/04, A61P 3/06, A61P 3/10, A61P 43/00, C07D 233/64, C07D 403/04, C12Q 1/02

(54) **HISTAMINE H3 AGONIST FOR USE AS THERAPEUTIC AGENT FOR LIPID/GLUCOSE METABOLIC DISORDER**

(30) Priority: 15.09.2005 JP 2005268227
(71) Applicant: BANYU PHARMACEUTICAL CO., LTD., Tokyo 102-8667 (JP)
(72) Inventor: YOSHIMOTO, Ryo, BANYU PHARMACEUTICAL CO., LTD., Tsukuba-shi Ibaraki 300-2611 (JP); ISHIHARA, Akane, BANYU PHARMACEUTICAL CO., LTD,, Tsukuba-shi Ibaraki 300-2611 (JP); KANATANI, Akio, BANYU PHARMACEUTICAL CO., LTD., Tsukuba-shi Ibaraki 300-2611 (JP); TOKITA, Shigeru, BANYU PHARMACEUTICAL CO., LTD., Tsukuba-shi Ibaraki 300-2611 (JP)
(74) Representative: Buchan, Gavin MacNicol
(86) International application number: PCT/JP2006/318548
(87) International publication number: WO 2007/032536

(57) **Abstract**

The present invention provides a histamine receptor H3 protein agonist (e.g. Imetit) used for anti-obesity or suppression of food intake. In addition, the present invention also provides a method for assaying a compound having a histamine receptor H3 protein as a target of drug discovery, and a compound obtained by the aforementioned assay method. According to the present invention, it becomes possible to provide a novel intended use of a histamine receptor H3 protein agonist for anti-obesity or suppression of food intake. Moreover, it also becomes possible to provide a method for assaying a compound having the aforementioned protein as a target of drug discovery, and a compound obtained by the aforementioned assay method.

## Description

### Technical Field

The present invention relates to a novel use of a histamine H3 agonist as a therapeutic agent for a lipid metabolic disease. In addition, the present invention relates to a method for assaying a compound effective for a lipid metabolic disease using histamine H3 as a target molecule.

### Background Art

Many hormones and neurotransmitters control the functions of a living body through specific receptor proteins existing on the cell membrane. A majority of such receptor proteins conduct intracellular signaling via activation of guanosine triphosphate-binding proteins (G proteins) coupled thereto. Thus, such receptor proteins are generically referred to as G protein-coupled receptor proteins.

Such G protein-coupled receptor proteins exist on the surface of cells or various functional cells of organs of a living body, and they play extremely important roles as targets of molecules that control the functions of the cells or organs of a living body, such as hormones, neurotransmitters, physiologically active substances, etc. Accordingly, G protein-coupled receptor proteins are attracting attention as target molecules in the development of pharmaceuticals.

It has been known that histamine is associated with inflammation. In addition, it has also been known that such histamine governs various vital functions such as eating behavior or the maintenance of energy (Non-Patent Document 1).

On the other hand, a histamine receptor H3 protein is one type of G protein-coupled receptor protein (Non-Patent Document 2). It has been reported that the amount of food intake of a test animal is reduced by administration of a histamine receptor H3 protein antagonist (Non-Patent document 3 and Patent Document 1). Furthermore, association of such a histamine receptor H3 protein with many physiological functions, such as association of the histamine receptor H3 protein with sleep (Non-Patent Document 4), has been suggested.
Patent Document 1: WO 9511894
Non-Patent Document 1: Prog. Neurobiol., Vol. 63, p. 637, 2001
Non-Patent Document 2: Mol. Pharmacol., Vol. 55, p. 1101, 1999
Non-Patent Document 3: Brain Res., Vol. 628, p. 235, 1993
Non-Patent Document 4: Neuropharmacology, Vol. 27, p. 111, 1988

### Disclosure of the Invention

However, under the current circumstances, the fact that the agonist of such a histamine receptor H3 protein has action against obesity and action to suppress food intake has not been known at all.

The present invention has been completed to solve the problems of the aforementioned prior art techniques. It is an object of the present invention to provide a novel use of a histamine receptor H3 protein agonist for anti-obesity and suppression of food intake. Moreover, it is another object of the present invention to provide a method for assaying a compound having the aforementioned protein as a target of drug discovery, and a compound obtained by the aforementioned assay method.

As a result of intensive studies direct towards the aforementioned objects, the present inventors have found that a histamine receptor H3 protein agonist has action against obesity and action to suppress food intake. Also, they have found that it is possible to do an assay of a compound having the aforementioned protein as a target of drug discovery (screening of the compound, for example), thereby completing the present invention.

That is to say, the histamine H3 agonist of the present invention is a therapeutic agent for a lipid/glucose metabolic disease.

Herein, as the above described histamine H3 agonist, a histamine H3 agonist comprising, as an active ingredient, a compound represented by the following Formula (I) or a pharmaceutically acceptable salt thereof is preferably used: 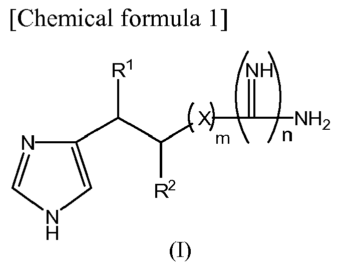 wherein R¹ and R² represent a hydrogen atom or a lower alkyl group, or when both m and n are 0, R¹ and R² represent the following (i) or (ii):
(i) R¹ and R² together form 5- or 6-membered aliphatic group; or
(ii) R¹ and the nitrogen atom of NH₂ in Formula (I) together form a 5- or 6-membered ring,
   X represents CH₂ or S, and
   m and n independently represent 0 or 1.

In addition, the histamine H3 agonist of the present invention preferably comprises, as an active ingredient, the compound represented by Formula (I), wherein both m and n are 0, or a pharmaceutically acceptable salt thereof.

Moreover, the histamine H3 agonist of the present invention preferably comprises, as an active ingredient, the compound represented by Formula (I), wherein both m and n are 1, or a pharmaceutically acceptable salt thereof.

Furthermore, the histamine H3 agonist of the present invention preferably comprises, as an active ingredient, the compound represented by Formula (I) or a pharmaceutically acceptable salt thereof, wherein the compound represented by Formula (I) is
1-amino-2-(1H-imidazol-4-yl)cyclopentane,
3-(1H-imidazol-4-yl)-2-butanamine,
2-methyl-3-(1H-imidazol-4-yl)pyrrolidine,
1-methyl-2-(1H-imidazol-4-yl)cyclohexylamine,
1H-imidazol-4-butanimidamide,
α-methylhistamine, or
carbamimidothioic acid 2-(1H-imidazol-4-yl)ethyl ester (Imetit).

Further, the histamine H3 agonist of the present invention is the therapeutic agent, wherein the above described lipid metabolic disease is obesity, eating disorder, diabetes, hyperlipidemia, hypercholesterolemia, or fatty liver.

Still further, the method of the present invention for assaying a compound is a method for assaying a compound used in the treatment of a lipid metabolic disease, comprising steps of: introducing a histamine H3 receptor gene into a cell, so as to prepare a cell that expresses a histamine H3 receptor; bringing a test compound into contact with the above described cell; and detecting the specific binding of the above described test compound to the above described histamine H3 receptor.

Still further, the histamine H3 agonist of the present invention is a method for assaying a compound used in the treatment of a lipid metabolic disease, comprising steps of: introducing a histamine H3 receptor gene into a cell, so as to prepare a cell that expresses a histamine H3 receptor; bringing a test compound into contact with the above described cell; measuring an activity of an intracellular signaling agent generated as a result of the contact; and comparing the thus measured activity with an activity of the above described intracellular signaling agent in a case where a test compound is not contacted with the above described cell.

Still further, the method of the present invention for assaying a compound is a method for assaying a compound used in the treatment of a lipid metabolic disease, comprising steps of: introducing a histamine H3 receptor gene into a cell, so as to prepare a cell that expresses a histamine H3 receptor; bringing a test compound into contact with the above described cell; measuring the expression level of the above described histamine H3 receptor or an intracellular signaling agent mediated by the above described histamine H3 receptor; and selecting a test compound that has increased or decreased the expression level of the above described histamine H3 receptor, when compared with a case where such a test compound is not contacted with the above described cell.

Still further, the method of the present invention for assaying a compound is a method for assaying a compound used in the treatment of a lipid metabolic disease, comprising steps of: bringing a test compound into contact with a histamine H3 receptor, and detecting a change in the activity of the histamine H3 receptor due to the contact.

Herein, in the methods for assaying a compound in the aforementioned four embodiments, the compound is preferably a histamine H3 agonist.

According to the present invention, it becomes possible to use a histamine receptor H3 protein agonist for the treatment of various lipid metabolic diseases, such as anti-obesity or suppression of food intake. In addition, it also becomes possible to provide a method for assaying a compound having the aforementioned protein as a target of drug discovery, and a compound obtained by the aforementioned assay method.

### Brief Description of the Drawings

**Figure 1** is a view showing the relationship between Imetit administration and the amount of food intake in the case of wild type mice. • (Black circle) indicates the results of control mice to which a solvent was administered, and o (white circle) indicates the results of mice to which Imetit was administered.
**Figure 2** is a view showing the relationship between Imetit administration and the amount of food intake in the case of H3RKO mice. • (Black circle) indicates the results of control mice to which a solvent was administered, and o (white circle) indicates the results of mice to which Imetit was administered.
**Figure 3** is a view showing Imetit administration and a change in body weight due to Imetit administration in the case of wild type mice. • (Black circle) indicates the results of control mice to which a solvent was administered, and o (white circle) indicates the results of mice to which Imetit was administered.
**Figure 4** is a view showing Imetit administration and a change in body weight due to Imetit administration in the case of H3RKO mice. • (Black circle) indicates the results of control mice to which a solvent was administered, and o (white circle) indicates the results of mice to which Imetit was administered.
**Figure 5** is a view showing a change in the amount of food intake by changing the amount of Imetit administered per body weight in the case of both wild type mice and H3RKO mice.
**Figure 6** is a view showing the relationship between Thioperamide administration and the amount of food intake in the case of both wild type mice and H3RKO mice.
**Figure 7** is a view showing a change in the amount of food intake by changing the concentration of Imetit administered in the case of both wild type mice and H3RKO mice.
**Figure 8** is a view showing a change in VO2 after administration of Imetit (vehicle: 5 mpk and 20 mpk).
**Figure 9** is a view showing a change in VCO2 after administration of Imetit (vehicle: 5 mpk and 20 mpk).
**Figure 10** is a view showing a change in RQ after administration of Imetit (vehicle: 5 mpk and 20 mpk).
**Figure 11** shows the mean values of VO2, VCO2, and RQ. The graphs show the results of control mice, mice to which 5 mpk Imetit was administered, and mice to which 20 mpk Imetit was administered, from the left.

### Best Mode for Carrying Out the Invention

Preferred embodiments of the present invention will be described in detail below.

The term "histamine receptor H3" is used in the present invention to mean a molecule having a seven-transmembrane structure that functions as a receptor of histamine H3. Organism species, which is the origin of such a histamine receptor H3, is not particularly limited. Examples of such organism species include a human, a monkey, a mouse, a rat, a canine, and a rabbit. Of these, a human histamine receptor H3 is preferable because the administration target of the histamine receptor H3 agonist of the present invention is a human and also because the assay target in assay of a compound is a human.

In addition, the histamine receptor H3 gene of the present invention includes a gene comprising a substitution, deletion, addition, or insertion of one or more nucleotides, as long as it has physiological functions equivalent to those of the histamine receptor H3 gene and it encodes a protein that functions as a histamine H3 receptor. Herein, the sequence of the aforementioned gene is not particularly limited, as long as it is a gene encoding the aforementioned protein. The sequence of the gene comprising such a substitution, deletion, addition, or insertion of nucleotides has homology of preferably 50% or more, more preferably 70% or more, further more preferably 80% or more, and particularly preferably 90% or more (for example, 91%, 92%, 93%, 94%, 95%, 96%, 97%, 98%, 99%, or more), with the sequence of the histamine receptor H3 gene of the present invention.

Moreover, the histamine receptor H3 gene of the present invention includes a nucleic acid that hybridizes with the histamine receptor H3 gene under stringent conditions. The term "hybridizes with ... under stringent conditions" is used herein to mean that two nucleic acid fragments mutually hybridize under hybridization conditions described in Molecular Cloning: A Laboratory Manual, 2nd edition, Cold Spring Harbor (1989), 9.47-9.62 and 11.45-11.61. More specifically, such hybridization under stringent conditions means that hybridization is carried out at approximately 45°C with 6.0 x SSC, and that the resultant is then washed at 50°C with 2.0 x SSC, for example. In order to select stringency, the salt concentration during the washing step can be ranged from approximately 2.0 x SSC and 50°C for low stringency, to approximately 0.2 x SSC and 50°C for high stringency. Moreover, the temperature during the washing step can be increased from a room temperature (approximately 22°C) for a low stringency condition, up to approximately 65°C for a high stringency condition.

Furthermore, the term "lipid/glucose metabolic disease" is used in the present invention to mean disease in general caused by the abnormity of lipid metabolism. Specific examples of such disease include obesity, eating disorder, diabetes, hyperlipidemia, hypercholesterolemia, and fatty liver.

### (1) Histamine receptor H3 agonist

The term "histamine receptor H3 agonist" is used in the present invention to mean a substance that causes intracellular signaling mediated by a histamine receptor H3. Such a histamine receptor H3 agonist is also referred to as a "histamine receptor H3 stimulant agent." Further, the molecular species of the histamine receptor H3 agonist of the present invention is not particularly limited, as long as it is a molecule that exhibits affinity for a histamine receptor H3 and functions as an agonist. Examples of such a molecular species include a low molecular weight compound, a protein, and a peptide. The type of the aforementioned low molecular weight compound is not particularly limited. Specific examples include a natural compound, an organic compound, and an inorganic compound. Specific examples of a histamine H3 receptor agonist used in the present invention include histamine, N-methylhistamine, N-,N-methylhistamine, N-ethylhistamine, N-propylhistamine, N-[2-(1H-imidazol-4-yl)ethyl]-pyrrolidine, (S),(S)-cyclopropylhistamine, (R),(R)-cyclopropylhistamine, SCH49647 (2S, 3R), SCH49648 (2R, 3S), SCH50971 (3R, 4R), immepip, VUF8621, VUF8973, SKF91606, and VUF4858.

Furthermore, another specific example of the histamine H3 agonist of the present invention is a compound represented by the following Formula (I) or a pharmaceutically acceptable salt thereof: 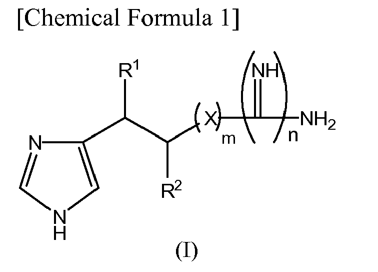 wherein R¹ and R² independently represent a hydrogen atom or a lower alkyl group, or when both m and n are 0, R¹ and R² represent the following (i) or (ii):
(i) R¹ and R² together form an aliphatic 5- or 6-membered ring; or
(ii) R¹ and the nitrogen atom of NH₂ in Formula (I) together form a 5- or 6-membered ring,
   X represents CH₂ or S, and
   m and n independently represent 0 or 1.

Hereafter, the compound represented by Formula (I) will be described.

The lower alkyl group represented by R¹ and R² means a linear or branched alkyl group containing 1 to 6 carbon atoms. Specific examples of such a lower alkyl group include methyl, ethyl, propyl, isopropyl, butyl, isobutyl, sec-butyl, tert-butyl and pentyl groups.

Specific examples of an aliphatic 5- or 6-membered ring formed by R¹ and R², when both m and n are 0, include cyclopentyl and cyclohexyl groups.

Specific examples of a 5- or 6-membered ring formed by R¹ and the nitrogen atom of NH₂, when both m and n are 0, include pyrrolidinyl and piperidinyl groups.

When R¹ and R² together form an aliphatic 5- or 6-membered ring, such R¹ and R² may bind to carbon atoms located at any of the termini, midpoints, and bases of the chains of the R¹ and R², so as to form the aforementioned aliphatic 5- or 6-membered ring.

When R¹ and the nitrogen atom of NH₂ in Formula (I) together form a 5- or 6-membered ring, the nitrogen atom may bind to a carbon atom located at any of the terminus, midpoint, and base of the chain of the R¹, so as to form the aforementioned 5- or 6-membered ring.

Moreover, when m is 0, the following Formula (II) indicates a single bond:

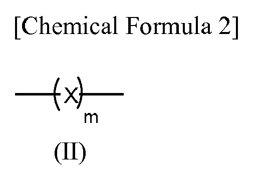

Furthermore, when n is 0, the following Formula (III) indicates a single bond:

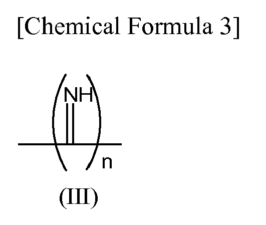

Among compounds represented by Formula (I), a compound wherein both m and n are 0, or a pharmaceutically acceptable salt thereof, and a compound wherein both m and n are 1, or a pharmaceutically acceptable salt thereof, are preferable.

It is to be noted, in a preferred embodiment, R¹, R², X, m and n in Formula (I) may be applied in any types of combinations.

More specific examples of the compound represented by Formula (I) include: a compound such as
1-amino-2-(1H-imidazol-4-yl)cyclopentane,
3-(1H-imidazol-4-yl)-2-butanamine,
2-methyl-3-(1H-imidazol-4-yl)pyrrolidine,
1-methyl-2-(1H-imidazol-4-yl)cyclohexylamine,
1H-imidazol-4-butanimidamide,
α-methylhistamine, or
carbamimidothioic acid 2-(1H-imidazol-4-yl)ethyl ester (Imetit); and a pharmaceutically acceptable salt thereof.

The compound represented by Formula (I) or a pharmaceutically acceptable salt thereof can be produced by the method described in EP 0420396; EP 0531219; J. Med. Chem., Vol. 38, p. 1593, 1995; or US 3,759,944, or by a method equivalent thereto, or by combing such methods with ordinary methods.

In addition, the compound represented by Formula (I) may be converted to a pharmaceutically acceptable salt or ester according to an ordinary method. On the contrary, conversion of such a salt or ester to a free compound may also be carried out according to an ordinary method.

Specifically, when the aforementioned compound represented by Formula (I) has a basic group derived from an amino group, a pyridyl group, etc. in the molecule thereof, the present compound is treated with an acid, so that it can be converted to a corresponding pharmaceutically acceptable salt.

Examples of such an acid addition salt include: hydrohalide salts such as hydrochloride, hydrofluoride, hydrobromide, or hydroiodide salt; inorganic acid salts such as nitrate, perchlorate, sulfate, phosphate, or carbonate; lower alkyl sulfonates such as methanesulfonate, trifluoromethanesulfonate, or ethanesulfonate; aryl sulfonates such as benzenesulfonate or p-toluenesulfonate; organic acid salts such as fumarate, succinate, citrate, tartrate, oxalate, or maleate; and acid addition salts that are organic acids including amino acids such as glutamate or aspartate.

Moreover, when the compound of the present invention has an acidic group in the group thereof, and when it has a carboxyl group, for example, the compound can also be converted to a corresponding pharmaceutically acceptable salt by treating it with a base. Examples of such a base addition salt include: alkaline metal salts such as sodium or potassium; alkaline-earth metal salts such as calcium or magnesium; ammonium salts; and organic base addition salts such as guanidine, triethylamine, or dicyclohexylamine.

Furthermore, the compound of the present invention may be present in the form of a free compound, or any given hydrate or solvate of the salt thereof. Further, on the contrary, conversion of a salt or ester to a free compound can also be carried out according to an ordinary method.

Still further, depending on the embodiment of the substituent thereof, the compound of the present invention may have stereoisomers such as optical isomers, diastereoisomers or geometrical isomers, or tautomers. Needless to say, such isomers are all included in the compound of the present invention. Further, needless to say, any given mixture of such isomers is also included in the compound of the present invention.

When the histamine receptor H3 agonist of the present invention is used as a medicine for humans or other animals, it is possible to produce a pharmaceutical preparation from the aforementioned substance according to a known pharmaceutical manufacturing method, as well as to directly administer such a substance to a patient. For example, the histamine receptor H3 agonist of the present invention is processed into a tablet, which is coated with sugar as necessary, a capsule, an elixir, or a microcapsule, and it can be orally used. Otherwise, the histamine receptor H3 agonist of the present invention is dissolved in water or other pharmaceutically acceptable solutions to produce a sterilized solution or suspension, and the thus produced sterilized solution or suspension can be parenterally used in the form of an injection. For example, the histamine receptor H3 agonist of the present invention is appropriately combined with a pharmaceutically acceptable carrier or medium, specifically, a sterilized water, a normal saline, vegetable oil, an emulsifier, a suspension, a surfactant, a stabilizer, a flavoring agent, an excipient, a vehicle, an antiseptic, a binder, etc., so that they can be mixed at a common unit dose form required for manufacturing a pharmaceutical preparation, thereby obtaining a pharmaceutical preparation.

Examples of an additive that can be mixed with a tablet or capsule include: binders such as gelatin, corn starch, gum tragacanth, or gum Arabic; excipients such as crystalline cellulose; a swelling agents such as corn starch, gelatin, or alginic acid; lubricants such as magnesium stearate; sweeteners such as sucrose, lactose, or saccharin; and flavoring agents such as peppermint, Gaultheria adenothrix oil, or cherry. When its unit dose form is a capsule, liquid carriers such as oil and fat may further be added to the aforementioned materials. A sterilized composition used for injection can be formulated according to a common pharmaceutical manufacturing method using a vehicle such as a distilled water for injection.

Examples of an aqueous solution used for injection include a normal saline and an isotonic solution comprising glucose or other auxiliary agents such as D-sorbitol, D-mannose, D-mannitol, or sodium chloride. Such an aqueous solution used for injection may also be used in combination with appropriate solubilizers including alcohols such as ethanol, polyalcohol such as propylene glycol or polyethylene glycol, or a nonionic surfactant such as polysorbate 80^{™} or HCO-50.

Examples of oily liquid include sesame oil and soybean oil. Such oily liquid may be used in combination with solubilizers such as benzyl benzoate or benzyl alcohol. In addition, such oily liquid may also be mixed with buffers such as a phosphate buffer or sodium acetate buffer, pain relief agents such as procaine hydrochloride, stabilizers such as benzyl alcohol or phenol, or antioxidants. The prepared injection solution is generally filled into a suitable ampule.

Such a pharmaceutical may comprise the compound of the present invention at a ratio of 1.0 to 100% by weight, and preferably 1.0 to 60% by weight.

A pharmaceutical preparation can be produced from the compound of the present invention according to the following formulation examples.

### (Formulation example 1)

10 parts of the compound of the below-described examples, 15 parts of heavy magnesium oxide, and 75 parts of lactose were uniformly mixed to produce powdered or fine-grained powder medicine with a size of 350 µm or smaller. This powder medicine was placed in a capsule vessel, so as to produce a capsule agent.

### (Formulation example 2)

45 parts of the compound of the below-described examples, 15 parts of starch, 16 parts of lactose, 21 parts of crystalline cellulose, 3 parts of polyvinyl alcohol, and 30 parts of distilled water were uniformly mixed. The mixture was subjected to crushing granulation, and it was then dried, followed by sieving, thereby producing a granule having a diameter between 1410 and 177 µm

### (Formulation example 3)

A granule was produced by the same method as in Formulation example 2. Thereafter, 3 parts of calcium stearate was added to 96 parts of the granule, and the mixture was then subjected to compression molding, so as to produce a tablet having a diameter of 10 mm.

### (Formulation example 4)

10 parts of crystalline cellulose and 3 parts of calcium stearate were added to 90 parts of the granule obtained by the method applied in Formulation example 2, and the obtained mixture was then subjected to compression molding, so as to produce a tablet having a diameter of 8 mm. Thereafter, a mixed suspension of syrup gelatin and sedimentation calcium carbonate was added to the aforementioned tablet, so as to produce a sugarcoated tablet.

When the compound of the present invention is used in clinical sites, the dosage and the number of doses are different depending on the sex, age and body weight of a patient, the degree of symptoms, the type and/or range of a therapeutic effect of interest, etc.

The compound of the present invention can be administered to patients according to a method known to persons skilled in the art, via an intranasal, transbronchial, intramuscular, percutaneous, or oral administration route, as well as via an intraarterial, intravenous, or subcutaneous injection. The dosage differs depending on the body weight or age of a patient, an administration method, etc. A suitable dosage can be selected, as appropriate, by persons skilled in the art. In addition, if the present agonist can be encoded by DNA, it is possible to incorporate such DNA into a gene therapy vector, so as to carry out gene therapy. The dosage and administration method differ depending on the body weight or age of a patient, symptoms, etc. A suitable dosage can be selected, as appropriate, by persons skilled in the art.

The dosage of the histamine receptor H3 agonist differs depending on symptoms. In the case of oral administration, it is generally considered to be between approximately 0.01 and 100 mg/kg, and preferably between approximately 0.03 and 1 mg/kg per adult per day.

In the case of parenteral administration, the dosage per administration differs depending on an administration target, a target organ, symptoms, and an administration method. For example, when the compound is administered in the form of an injection, it is considered convenient to administer it at a dosage generally between approximately 0.001 and 10 mg/kg, and preferably between approximately 0.001 and 0.1 mg/kg, per adult per day, via an intravenous injection.

Ordinary internists, veterinarians, or clinicians can easily determine an effective amount of drug necessary for inhibiting, suppressing, or terminating progression of disease.

### (2) Assay of compound

It is possible to use a histamine receptor H3 gene or protein to assay a compound acting on a histamine receptor H3. Methods for detecting the action of a test compound on the aforementioned histamine receptor H3 include: a method for detecting the specific binding of a test compound to the aforementioned receptor; a method for detecting the expression level of the gene that has been changed due to contact with such a test compound; and a method for detecting an intracellular signaling activity mediated by a histamine receptor H3 generated as a result of such contact. These methods will be successively explained below.

First, a first method of the present invention for assaying a compound will be described.

The first method of the present invention for assaying a compound comprises steps of: introducing a histamine H3 receptor gene into a cell, so as to prepare a cell that expresses a histamine H3 receptor; bringing a test compound into contact with the above described cell; and detecting the specific binding of the above described test compound to the above described histamine H3 receptor.

The type of a test compound is not particularly limited. Examples of such a test compound include single compounds such as a natural compound, an organic compound, an inorganic compound, a protein or a peptide, a compound library, expression products derived from a gene library, cell extracts, cell culture supernatants, products from fermentation microorganisms, marine organism extracts, plant extracts, prokaryotic cell extracts, eukaryotic unicellular extracts, and animal cell extracts. The aforementioned test compound may be labeled, as necessary, before use. Examples of such a labeling method include radiolabeling and fluorescent labeling. Moreover, the aforementioned test compound also includes a mixture obtained by mixing several types of such test compounds.

Cells that express such histamine receptor H3 genes may be prepared by persons skilled in the art according to a known method. The type of a specific method for preparing such cells is not particularly limited. The following method may be applied, for example. That is, a histamine receptor H3 gene or a nucleic acid comprising the portion thereof is cloned into an expression vector comprising a preferred promoter and transcriptional regulatory elements. The vector having the cloned nucleic acid is then introduced into a host cell, so as to prepare a cell that expresses the histamine receptor H3 gene. The type of the aforementioned vector is not particularly limited herein, as long as it is available as an expression vector. Examples of such an expression vector include pCMV-Tag, pcDNA3.1, pBlueBacHis2, pCI-neo, pcDNAI, pMC1neo, pXT1, pSG5, pEF1/V5-HisB, pCR2.1, pET11, λgt11, and pCR3.1.

Subsequently, the expression vector, into which a histamine receptor H3 gene or a nucleic acid comprising the portion thereof has been introduced, is introduced into a host cell. The type of such a host cell is not particularly limited, as long as it is generally used in expression of a gene. Any one of an animal cell, an insect cell, a plant cell, and a microorganism may be used. Specific examples of such a host cell include COS1, COS7, CHO, NIH/3T3, 293, Raji, CV11, C1271, MRC-5, CPAE, HeLa, 293T, and Sf9. The type of a method for introducing such an expression vector into a host cell is not particularly limited, as long as it is a known method. Specific examples of such a method include electroporation, a calcium phosphate method, a DEAE-dextran method, a lipofection method, and use of a gene gun.

Thereafter, a test compound is contacted with the thus prepared cell that expresses a histamine receptor H3. The type of a method for bringing a test compound into contact with the aforementioned cell is not particularly limited. For example if such a histamine receptor H3 has been purified, such a contact can be carried out by adding a test sample to the purified sample. In addition, if such a histamine receptor H3 is in a state where it is expressed in a cell (including the receptor that is expressed on a cell membrane) or in a state where it is expressed in a cell extract, such a contact can be carried out by adding a test sample to a cell culture solution or the aforementioned cell extract, respectively. When such a test sample is a protein, such a contact can be carried out by introducing a vector comprising DNA encoding the aforementioned protein into a cell in which the histamine receptor H3 has been expressed, or by adding the aforementioned vector to a cell extract in which the histamine receptor H3 has been expressed, for example.

The binding of a receptor that is expressed on the surface of a cell to a test compound can be detected using a labeling substance attached to the bound compound (e.g. detection of the binding amount based on radioactivity or fluorescence intensity), or using, as an indicator, intracellular signaling generated as a result of the binding of a test compound to the aforementioned receptor on the surface of a cell (e.g. activation of a G protein, a change in cAMP or Ca²⁺ concentration (FLIPR (Fluorometric Imaging Plate Reader), etc. can be used), activation of phospholipase C, pH change, and internalization of the receptor).

Next, second and third methods of the present invention for assaying a compound will be described.

The second method of the present invention for assaying a compound comprises steps of: introducing a histamine H3 receptor gene into a cell, so as to prepare a cell that expresses a histamine H3 receptor; bringing a test compound into contact with the above described cell; measuring an activity of an intracellular signaling agent generated as a result of the contact; and comparing the thus measured activity with an activity of an intracellular signaling agent in a case where a test compound is not contacted with the above described cell.

Moreover, the third method of the present invention for assaying a compound comprises steps of: introducing a histamine H3 receptor gene into a cell, so as to prepare a cell that expresses a histamine H3 receptor; bringing a test compound into contact with the above described cell; measuring the expression level of the above described histamine H3 receptor or an intracellular signaling agent mediated by the above described histamine H3 receptor; and selecting a test compound that has increased or decreased the expression level of the above described histamine H3 receptor or the intracellular signaling agent mediated by the above described histamine H3 receptor, when compared with a case where such a test compound is not contacted with the above described cell.

In the second and third methods for assaying a compound, a method for preparing a cell that expresses a histamine receptor H3 gene and a method for bringing a test compound into contact with the aforementioned cell are the same as those in the first method for assaying a compound.

In the second and third methods for assaying a compound, the expression level or activity of a molecule (including a histamine receptor H3) existing on an intracellular signaling pathway generated by bringing a test compound into contact with the cell that expresses the histamine H3 receptor can be used as an indicator. Herein, when the aforementioned expression level is used as an indicator, the type of a method for measuring such an expression level is not particularly limited. Examples of such a measurement method include Northern blotting, Western blotting, and use of a DNA chip. The term "expression level" is used herein in the present invention to mean the absolute or relative amount of the transcriptional product of a gene encoding a protein existing on a signaling pathway mediated by the histamine receptor H3. In this case, the aforementioned gene includes either DNA or mRNA. In addition, when a detection target of expression is a protein, the "expression level" of the protein means the absolute or relative amount of the translated product of a protein existing on a signaling pathway mediated by the histamine receptor H3. Moreover, when the activity of a molecule existing on an intracellular signaling pathway is used as an indicator, the type of method for measuring such activity is not particularly limited. A preferred method may be selected depending on the type of a molecule as a measurement target.

Next, a fourth method of the present invention for assaying a compound will be described.

The fourth method of the present invention for assaying a compound comprises steps of: bringing a test compound into contact with a histamine H3 receptor; and detecting a change in the activity of the histamine H3 receptor due to the contact.

The type of a method for bringing a test compound into contact with a histamine H3 receptor is not particularly limited. Specific methods include: a method of mixing a test compound with a histamine H3 receptor in a solution such as a buffer (a phosphate buffer, etc.), so as to bring the test compound into contact with the histamine H3 receptor; and a method of immobilizing a histamine receptor H3 protein on a membrane and then bringing the aforementioned receptor into contact with a test compound on the membrane.

Thereafter, a change in the activity of the histamine receptor H3 generated as a result of the contact is detected.

A method for measuring the activity of a protein may be determined, as appropriate, depending on the properties of the used protein. Specifically, a method using the binding activity of a ligand to the histamine receptor H3 as an indicator is applied, for example.

The type of the aforementioned method using the binding activity of a ligand as an indicator is not particularly limited. Specifically, a method of measuring the affinity of a test compound for a membrane, on which a histamine receptor H3 has been immobilized, so as to measure the binding activity of the ligand, is applied, for example. The test compound used herein may be labeled with a radioisotope or the like in order to facilitate detection. Furthermore, as the aforementioned method of detecting a binding activity, a method for detecting a compound binding to a histamine receptor H3 by competing with a ligand labeled with a radioisotope is also applied. When such a method is applied, the labeling of a test compound is unnecessary.

As described above, the activity and/or pharmacological effect of a compound are detected according to the method of the present invention for assaying a compound. As a result, when a binding activity in the presence of a test compound is lower than a binding activity in the absence of a test compound (control), it is determined that the test compound has an activity of inhibiting the binding of the histamine receptor H3 of the present invention to a ligand. Such a compound includes a compound having an activity of binding to the aforementioned receptor and inducing intracellular signaling (an agonist) and a compound that does not have the aforementioned activity (an antagonist). Such an agonist has a physiological activity equivalent to those of a ligand binding to the aforementioned receptor and an analogue thereof. On the other hand, such an antagonist suppresses the physiological activities of the ligand binding to the aforementioned receptor and an analogue thereof. Accordingly, such an agonist and an antagonist are useful as pharmaceutical compositions for treating a disease caused by the abnormity of such a signaling system mediated by the histamine receptor H3 of the present invention.

Moreover, by the method of the present invention for assaying a compound, a substance that promotes or inhibits intracellular signaling after a test compound has bound to the histamine receptor H3 can be screened. That is to say, by assaying several test compounds according to the aforementioned methods, a compound that functions as an agonist or antagonist can be selected. As a result of such selection, when a change in intracellular signaling obtained in a case where a ligand and an analogue thereof are allowed to act on the receptor in the presence of a test compound is compared with a change in intracellular signaling obtained in a case where a ligand and an analogue thereof are allowed to act on the receptor in the absence of the test compound, if such a change in suppressed, it is determined that the test compound is a compound that inhibits intracellular signaling after the test compound has bound to the histamine receptor H3. To the contrary, if the test compound enhances intracellular signaling, it is determined that the test compound is a compound that promotes intracellular signaling after the test compound has bound to the histamine receptor H3. A compound selected by such a screening method is effective for treating a lipid metabolic disease or alleviating the symptoms thereof. Among others, a compound that functions as a histamine receptor H3 agonist is effective particularly for treating a lipid metabolic disease or alleviating the symptoms thereof.

### Examples

The present invention will be more specifically described in the following examples. However, these examples are not intended to limit the scope of the present invention.

### Animal

Histamine H3 receptor knockout mice (hereinafter referred to as "H3RKO mice") were produced by the method of Takahashi et al. (J. Clin. Invest, Vol. 110, p. 1791, 2002). The obtained knockout mice were subjected to back crossing with C57BL/6J over 5 generations. Six- to nine-month-old male litters were used in an acute drug administration test. On the other hand, 18- to 20-month-old mice whose parents were H3RKO mice or wild type mice (N5 of C57BL/6J) were used in a chronic drug administration test.

All mice were individually bred at 25°C, and light and dark were controlled every 12 hours. During breeding, water and feed were freely given to the mice. As such feed, CE2 (CLEA Japan, Inc.) was given to thin mice, and medium high fat diet (Oriental Bio Service Co., Ltd.) was given to DIO mice.

### Compound

Each of Imetit (Tocris) and Thioperamide (Sigma-Aldrich) was dissolved in 0.5% methylcellulose for oral administration. The concentration of each compound was set at 5 ml/kg.

### Measurement of amount of food intake

Imetit or Thioperamide was administered to the mice at a time from 17:00 to 18:00. The amount of food intake was measured based on the weight of feed at 19:00 and 23:00.

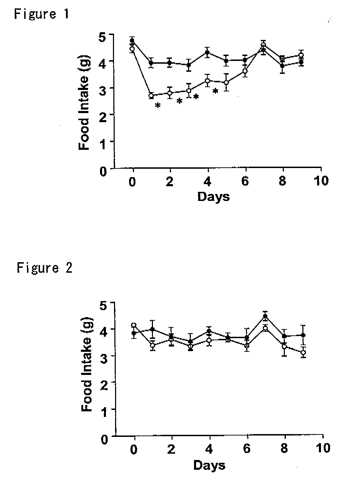

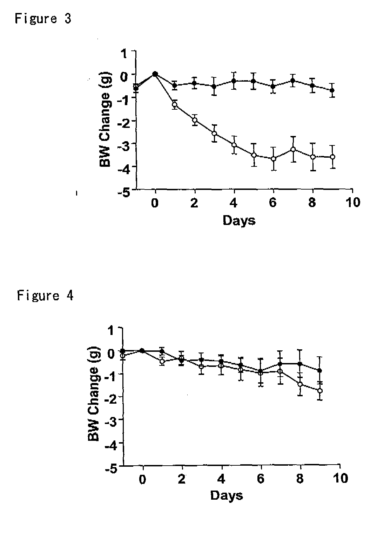

As shown in Figure 1, it was confirmed that the amount of food intake in the case of Imetit administration mice was lower than that of control mice. In addition, as shown in Figure 2, when the same test was carried out on H3RKO mice, such a decrease in the amount of food intake was not observed even in the case of administering Imetit. These results demonstrated that action of Imetit suppresses food intake mediated by a histamine H3 receptor. With regard to these mice, a change in body weight due to Imetit administration was measured. As shown in Figure 3, when Imetit was administered to wild type mice, their body weight was decreased depending on the number of doses. On the other hand, as shown in Figure 4, when Imetit was administered to H3RKO mice, such a change in body weight was not observed. Thus, it was confirmed that Imetit acts to suppress body weight increase, mediated by a histamine H3 receptor.

### Anti-obesity test

Mice were divided into groups, depending on mean body weight and the amount of daily food intake before administration of the compound. The body weight and the amount of food intake were measured every day.

In the test using Thioperamide, mice were divided into 4 groups: a group in which a solvent was administered to C57BL/6J DiO mice at 9:00; a group in which 10 mpk Thioperamide was administered thereto at 9:00; a group in which a solvent was administered to C57BL/6J DiO mice at 17:00; and a group in which 10 mpk Thioperamide was administered thereto at 17:00. On the other hand, in the test using Imetit, a solvent or Imetit was administered to wild type mice or H3RKO mice twice a day. On the 10^{th} day after the administration, such mice were left in a starvation state for 2 hours. Thereafter, blood was collected from the orbit of each mouse, and the glucose and insulin levels were measured.

A change in body weight was calculated by dividing a decrease in body weight by the body weight on day 0 at initiation of the test, and the calculated value was expressed as a percentage. The degree of obesity was measured using an NMR analyzer (Minispec; Bruker).

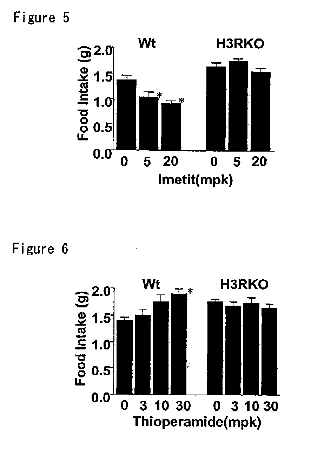

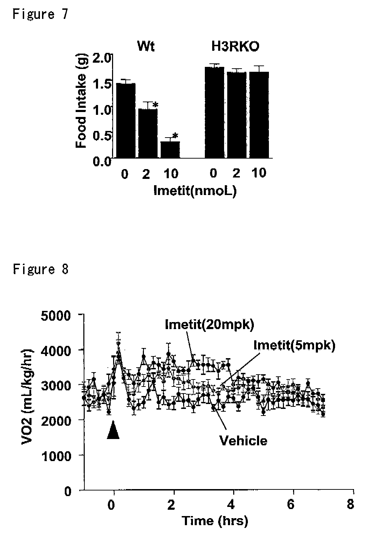

As shown in Figure 6, there was a tendency that the body weight of fat mice was increased by administration of Thioperamide, depending on the number of doses. Moreover, as shown in Figures 5 and 7, the body weight of fat mice was significantly decreased by administration of Imetit. Furthermore, as shown in Table 1, the body weight-decreasing effect of Imetit depends on adipose tissues, and such effect was not observed in H3RKO mice. Thus, it was found that the body weight-decreasing effect of Imetit is an H3 selective effect. Further, an insulin concentration in blood was decreased together with a fat-decreasing effect. Thus, it was found that abnormal glucose tolerance was also improved.

**Table 1**

| | Treatment | Fat (%) | Lean body mass (%) | Glucose (mg/dL) | Insulin (ng/mL) |
|---|---|---|---|---|---|
| Wild type | Solvent administration | 45.9 ± 0.9 | 53.2 ± 0.9 | 169.0 ± 9.1 | 7.5 ± 0.9 |
| | Imetit administration | 39.2 ± 2.0* | 58.3 ± 1.6* | 164.1 ± 6.4 | 4.7 ± 0.8* |
| H3RKO | Solvent administration | 47.6 ± 2.0 | 51.4 ± 1.5 | 160.9 ± 9.7 | 6.0 ± 1.5 |
| | Imetit administration | 46.3 ± 2.0 | 52.7 ± 1.4 | 166.7 ± 10.5 | 4.7 ± 0.9 |

| | | | | | |
|---|---|---|---|---|---|
| In the table, * indicates p < 0.05 (Student's t-test) to a solvent administration control. | | | | | |

### Measurement of energy consumption

Energy consumption was measured using Indirect calorimetry (Columbus Instrument). That is to say, C57BL/6J mice were acclimatized in a test chamber overnight, and VO2 (oxygen consumption) and VCO2 (glycogenesis) were then measured for 1 minute every 10 minutes. Sample air was collected every 10 minutes. RQ (respiratory quotient) was calculated by dividing VO2 by VCO2. The test was carried out from 9:00 to 17:00. During the test, mice were allowed to freely ingest feed and water.

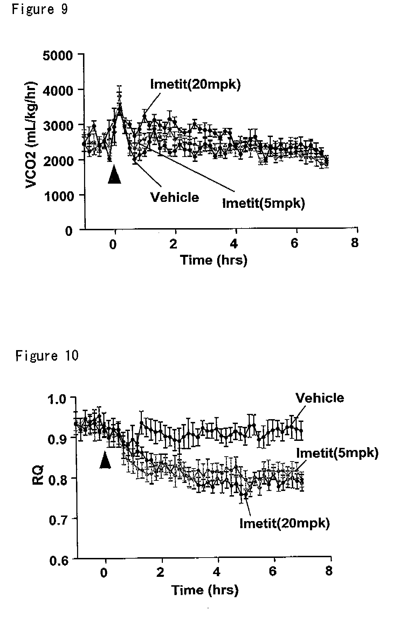

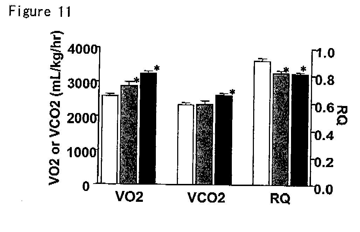

As shown in Figures 8 to 11, increases in VO2 (Figure 8) and VCO2 (Figure 9) and a decrease in RO (Figure 10) were observed as a result of the administration of Imetit. These results demonstrated that Imetit promotes energy metabolism, and in particular that it bums fat.

### Industrial Applicability

According to the present invention, it becomes possible to use a histamine receptor H3 protein agonist for the treatment of various lipid metabolic diseases, such as anti-obesity or suppression of food intake. In addition, it also becomes possible to provide a method for assaying a compound having the aforementioned protein as a target of drug discovery, and a compound obtained by the aforementioned assay method.

## Claims

1. A histamine H3 agonist which is a therapeutic agent for a lipid/glucose metabolic disease.

2. The histamine H3 agonist according to claim 1, which comprises, as an active ingredient, a compound represented by the following Formula (I) or a pharmaceutically acceptable salt thereof: 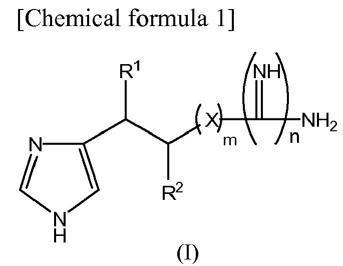 wherein R¹ and R² represent a hydrogen atom or a lower alkyl group, or when both m and n are 0, R¹ and R² represent the following (i) or (ii):
(i) R¹ and R² together form 5- or 6-membered aliphatic group; or
(ii) R¹ and the nitrogen atom of NH₂ in Formula (I) together form a 5- or 6-membered ring,
X represents CH₂ or S, and
m and n independently represent 0 or 1.

3. A histamine H3 agonist, which comprises, as an active ingredient, the compound according to claim 2, wherein both m and n are 0, or a pharmaceutically acceptable salt thereof.

4. A histamine H3 agonist, which comprises, as an active ingredient, the compound according to claim 2, wherein both m and n are 1, or a pharmaceutically acceptable salt thereof.

5. A histamine H3 agonist, which comprises, as an active ingredient, the compound represented by Formula (I) or a pharmaceutically acceptable salt thereof, wherein the compound represented by Formula (I) is
1-amino-2-(1H-imidazol-4-yl)cyclopentane,
3-(1H-imidazol-4-yl)-2-butanamine,
2-methyl-3-(1H-imidazol-4-yl)pyrrolidine,
1-methyl-2-(1H-imidazol-4-yl)cyclohexylamine,
1H-imidazol-4-butanimidamide,
α-methylhistamine, or
carbamimidothioic acid 2-(1H-imidazol-4-yl)ethyl ester (Imetit).

6. The histamine H3 agonist according to any one of claims 1 to 5, wherein said lipid metabolic disease is obesity, eating disorder, diabetes, hyperlipidemia, hypercholesterolemia, or fatty liver.

7. A method for assaying a compound used in the treatment of a lipid metabolic disease, comprising steps of:
introducing a histamine H3 receptor gene into a cell, so as to prepare a cell that expresses a histamine H3 receptor;
bringing a test compound into contact with said cell; and
detecting the specific binding of said test compound to said histamine H3 receptor.

8. A method for assaying a compound used in the treatment of a lipid metabolic disease, comprising steps of:
introducing a histamine H3 receptor gene into a cell, so as to prepare a cell that expresses a histamine H3 receptor;
bringing a test compound into contact with said cell;
measuring an activity of an intracellular signaling agent generated as a result of the contact; and
comparing the thus measured activity with an activity of the intracellular signaling agent in a case where a test compound is not contacted with said cell.

9. A method for assaying a compound used in the treatment of a lipid metabolic disease, comprising steps of:
introducing a histamine H3 receptor gene into a cell, so as to prepare a cell that expresses a histamine H3 receptor;
bringing a test compound into contact with said cell;
measuring an expression level of the histamine H3 receptor or an intracellular signaling agent mediated by the histamine H3 receptor; and
selecting a test compound that has increased or decreased the expression level of the histamine H3 receptor or the intracellular signaling agent mediated by the histamine H3 receptor, when compared with a case where such a test compound is not contacted with said cell.

10. A method for assaying a compound used in the treatment of a lipid metabolic disease, comprising steps of:
bringing a test compound into contact with a histamine H3 receptor; and
detecting a change in the activity of the histamine H3 receptor due to the contact.

11. The method for assaying a compound according to any one of claims 7 to 10, wherein said compound is a histamine H3 agonist.

12. A histamine H3 agonist, wherein the agonist is isolated by the method for assaying a compound according to any one of claims 7 to 11.
